# EUROPÄISCHE PATENTSCHRIFT

(11) **EP 2 885 620 B1**
(45) Veröffentlichungstag und Bekanntmachung des Hinweises auf die Patenterteilung: **08.07.2020**
(21) Anmeldenummer: 13744438.6
(22) Anmeldetag: 27.07.2013
(51) Int. Cl.: G01N 1/34, G01N 15/06, G01N 33/28

(54) **VORRICHTUNG ZUM FESTSTELLEN VON PARTIKELVERSCHMUTZUNGEN IN FLUIDEN**
DEVICE FOR DETERMINING PARTICULATE CONTAMINATION IN FLUIDS
DISPOSITIF DE DÉTERMINATION DE CONTAMINANTS PARTICULAIRES DANS DES FLUIDES

(30) Priorität: 17.08.2012 DE 102012016458
(43) Veröffentlichungstag der Anmeldung: 24.06.2015
(73) Patentinhaber: Hydac Filter Systems GmbH, 66280 Sulzbach/Saar (DE)
(72) Erfinder: KLEBER, Jörg, 66538 Neunkirchen (DE)
(74) Vertreter: Bartels und Partner, Patentanwälte
(86) Internationale Anmeldenummer: PCT/EP2013/002231
(87) Internationale Veröffentlichungsnummer: WO 2014/026733

(56) Entgegenhaltungen:
- WO-A1-2008/074559
- DE-A1- 3 931 497
- DE-A1- 19 628 690
- DE-A1-102009 024 561
- JP-A- S62 255 849
- US-A- 5 968 371

## Beschreibung

Die Erfindung betrifft eine Vorrichtung zum Feststellen von Fremdstoffen wie Partikeln in Fluiden, die neben diesem Partikeleintrag weitere fluidfremde Verunreinigungen, wie feste, flüssige und gasförmige Fremdstoffe, aufweisen können, mit den Merkmalen im Oberbegriff von Anspruch 1.

Der Begriff "Fluide" im vorliegenden erfindungsgemäßen Sinne meint Druckflüssigkeiten, insbesondere in Form von Hydraulikölen jedweder Art. Dahingehende Druckflüssigkeiten stellen jeweils ein wichtiges Element in sogenannten tribologischen Systemen dar. Dabei besteht ein tribologisches System regelmäßig aus mindestens zwei Werkstoff-Flächen, deren Reibung durch einen Schmierstoff, hier die Druckflüssigkeit, vermindert wird. Obwohl dahingehende Druckflüssigkeiten heutzutage einen sehr hohen qualitativen Standard aufweisen und neben dem Verhindern von Korrosion, die Reibung zwischen den genannten Werkstoffflächen minimieren, sofern sie eine Relativbewegung zueinander ausüben, und dabei auch den Verschleiß an diesen Flächen vermeiden helfen, kommt es dennoch zumindest langfristig gesehen durch abrasive Verschleißvorgänge ungewollt zu einem Partikeleintrag in das Druckfluid, dessen vorstehend bezeichneten Eigenschaften darunter leiden. Und obwohl, um einen wartungsarmen Betrieb von hydraulischen Anlagen gewährleisten zu können, aufwendige Filtersysteme zum Einsatz kommen, zum Abfiltrieren der Partikelverschmutzung aus dem Druckfluid, sind hier Grenzen gesetzt und insbesondere kann es bei einem Versagen des jeweiligen Filtersystems, beispielsweise durch "Verblocken" seiner Filterelemente und Öffnen von Bypass-Einrichtungen zum Umgehen des jeweiligen durch Verblocken betroffenen Filterelementes des Filtersystems, zu einem vermehrten Partikeleintrag mit festen Verschleißstoffen kommen, die ein Stilllegen der gesamten hydraulischen Anlage nebst Austauschen des Druckfluides (Hydrauliköl) als notwendig erscheinen lassen.

Um einen insbesondere vermehrten Partikeleintrag in das Druckfluid feststellen zu können, dienen sogenannte Partikelsensoren, die vorrangig optisch oder elektronisch (induktiv) zumindest die Anzahl der Partikel im Fluid über eine geeignete Messwerteelektronik feststellen können, um dergestalt eine Aussage über die jeweils herrschende Qualität des Druckfluids zu treffen.

Unter dem angesprochenen optischen Messverfahren wird die elektronische Analyse durch eine Lichtabschattung angesprochen, die fachsprachlich auch als Extinktionsverfahren bezeichnet wird. Diese elektronische Analyse zählt die Partikel über einen optischen Sensor. Dabei wird der Lichtstrahl des optischen Sensors je nach Größe der Partikel mehr oder weniger abgeschattet. Die Differenz zwischen der abgesandten Lichtleistung und der in einer Fotodiode aufgefangenen Lichtleistung unter Einbezug der Absorption im Fluidmedium kann dann als Maß für die Größe des Partikels herangezogen werden. Die bekannte Messmethode kann online erfolgen, so dass vor Ort die Messergebnisse dann direkt vorliegen.

Andere moderne Partikelsensoren, wie sie beispielsweise in der WO 2007/ 088015 A1 aufgezeigt sind, erlauben derart eine Erhöhung der Empfindlichkeit hinsichtlich der festzustellenden oder zu detektierenden Partikel, dass nicht nur sehr kleine Partikel ohne Weiteres detektiert werden können, sondern dass neben der eigentlichen Anzahl an auftretenden Feststoff-Partikeln in einer vorgebbaren Volumeneinheit des Druckfluids auch deren Partikelgröße sich bestimmen lässt. Die bekannte Sensoreinrichtung nach der genannten WO-Veröffentlichung setzt hierfür einen induktiven Partikelzähler ein, wobei die Vorrichtung mindestens eine Feldspule zum Erzeugen eines den Fluidstrom mindestens abschnittsweise abdeckenden Magnetfelds aufweist und eine Sensorspule hat, die mit einer elektronischen Auswerteeinrichtung verbunden, mittels dem aus dem in der Sensorspule induzierten Signal die Anwesenheit eines Partikels in den Fluidstrom im skizzierten Rahmen feststellt oder detektiert.

Voraussetzung für die vorstehend beschriebenen Partikelfeststell- oder Messverfahren ist eine Kalibrierung des Gerätes anhand von Testpartikeln (fachsprachlich auch als Teststaub bezeichnet). Während frühere Kalibrierungen als Partikelgröße die längste Ausdehnung der Testpartikel eingesetzt haben, definieren neuere Kalibrierverfahren als Partikelgröße den mittleren Durchmesser eines flächengleichen Kreises, der den jeweils vorgegebenen Testpartikel randseitig umschließt.

Als weiterer Fremdstoff in Druckflüssigkeiten wie Hydraulikölen kommt regelmäßig Luft vor, wobei die Luft in der Druckflüssigkeit gelöst ist, als Luft in Form einer Öl-Dispersion vorliegt oder auf dem Fluid eine Art Oberflächenschaum ausbildet, wie man ihn regelmäßig in Vorratstanks von hydraulischen Anlagen findet. Die im Druckfluid gelöste Luft unter einem vorgebbaren Betriebsdruck des Fluids verursacht regelmäßig keine Probleme wie Betriebsstörungen der hydraulischen Anlagen. Erst wenn vermehrt Luft von außen zusätzlich in das Druckfluid gelangt oder die in Druckfluid gelöste Luft durch Druckentspannungsvorgänge, beispielsweise auf der Ableitungsseite von hydraulischen Ventilen oder im Hydrauliktank, meist fein dispergiert im Öl als Luftbläschen austritt, kann es zu Betriebsstörungen kommen, da das dann entstehende Öl-Luft-Gemisch kompressibel wird, insbesondere an heißen Stellen innerhalb des Hydraulikkreises. Im Übrigen wird dergestalt auch nachteilig die Ausbildung des hydrodynamischen Schmierfilms an den zu schmierenden Bauteilen beeinflusst. In ölführenden Systemen können dann oft erhebliche Störungen auftreten, insbesondere in Bereichen von schnelllaufenden Getrieben und Kompressoren, wie sie in der Windkraftanwendung zum Einsatz kommen.

Neben den gasförmigen Fremdstoffen wie den angesprochenen Luftblasen tritt als weiterer Fremdstoff im Fluid regelmäßig Wasser in flüssiger Form auf, beispielsweise als Kondensat bedingt durch Abkühlvorgänge der hydraulischen Anlage. Neben den Feststoffen stellt Wasser die gefährlichste Verunreinigung in Schmier- und Hydrauliksystemen dar, da diese sowohl als Mineral- als auch als Synthetiköl einen temperaturabhängigen Wassersättigungspunkt haben, der bei Überschreiten dazu führt, dass freies Wasser (emulgiert) auftritt. Wasser in Hydrauliksystemen fördert aber die Korrosion der Komponenten und erhöht die Gefahr der Kavitation von Hydraulikpumpen. Des Weiteren können die in den Fluiden vorhandenen Additive, um der Ölalterung zu begegnen, mit dem freien Wasser reagieren mit der Folge, dass sich Oxide, saure Schlämme sowie Harze ausbilden, was insgesamt zur "Verschlammung" des hydraulischen Gesamtsystems mit beiträgt.

Des Weiteren treten im Druckfluid Alterungsprodukte (Varnish) auf, die sich aus der Oxidation des Druckfluids unter Luftsauerstoff ergeben. Hohe Betriebstemperaturen begünstigen die dahingehenden Alterungsprozesse im Öl. Die Alterung eines Öls beginnt in der Regel zunächst sehr langsam, insbesondere wenn Oxidationsinhibitoren (Additive) diesen Prozess bremsen. Die Inhibitoren (Additive) fangen die reaktionsfreudigen Moleküle (Radikale) im Öl ein und neutralisieren die sauerstoffhaltigen Verbindungen. So wird das Druckfluid vor dem schnell zunehmenden Angriff durch die Additive geschützt. Sind diese jedoch verbraucht, läuft die Alterung des Öls ungebremst und damit sehr schnell ab. Das Endprodukt der Ölalterung sind dann jeweils ölunlösliche (Ölschlamm) sowie saure Substanzen, die die Korrosion von Bauteilen im gesamten hydraulischen Kreis erhöhen.

Bei der üblichen, vorstehend beschriebenen Partikelfeststellung oder Partikeldetektion gelangen all diese Fremdstoffe, die teilweise auch filtergängig sind, d. h. die ungestört ein Filtersystem passieren können, ohne von den Filtermedien aus dem Fluidstrom herausgefiltert zu werden, zu dem jeweiligen Partikelsensor (optisch, induktiv etc.), um dort als Partikel, also als Feststoffverschmutzung detektiert zu werden mit der Folge, dass der Partikelsensor nebst angeschlossener Auswerteelektronik die tatsächlich vorliegende Partikelverschmutzung im Druckfluid überhaupt nicht mehr feststellen kann. Dies führt dann zu Falschaussagen über den Partikeleintrag an Feststoffen im Druckfluid und gegebenenfalls zu einem verfrühten Austausch des in insoweit noch gar nicht unbrauchbar gewordenen Öls. Insbesondere wird beim Auftreten ständig hoher Partikelverschmutzung in Form von Feststoffen ein Durchschnittsfachmann auf dem Gebiet von Hydrauliksystemen Überlegungen anstellen, wie er durch eine qualitativ verbesserte Komponentenauswahl, insbesondere der zu schmierenden, bewegenden Maschinenteile einen erhöhten Feststoff-Partikeleintrag vermeiden kann, obwohl, wie dargelegt, dies gar nicht notwendig ist, weil die detektierte Verschmutzung nicht von den Feststoffpartikeln als dem einen Fremdstoff herrührt, sondern von den sonstigen genannten weiteren Fremdstoffen in fester Form (Ölalterungsprodukte), in flüssiger Form (Wasser) und/oder in gasförmiger Form (Luftblasen).

Die DE 10 2009 024 561 A1 beschreibt eine Vorrichtung zum Feststellen von Fremdstoffen, wie Partikeln, in Fluiden, die neben diesem Partikeleintrag weitere fluidfremde Verunreinigungen, wie feste, flüssige und gasförmige Fremdstoffe, aufweisen können, mit einer Mess-Strecke, die einen Partikelsensor zum Feststellen des Partikeleintrages aufweist, wobei eine Probenaufbereitungs-Strecke mittels einer Anschlusseinrichtung fluidführend an die Mess-Strecke anschließbar ist, wobei die Probenaufbereitungs-Strecke zumindest eine zusätzliche Einrichtung zum Feststellen der jeweils weiteren fluidfremden Verunreinigung und/oder zu deren zumindest teilweisem Ausscheiden aus dem Fluid aufweist, wobei die Anschlusseinrichtung aus elektromagnetisch ansteuerbaren Schaltventilen, insbesondere in Form von 3/2-Wege-Ventilen, gebildet ist, und wobei zwischen den zwei Schaltventilen der Anschlusseinrichtung eine Baugruppe bestehend aus einer Motor-Pumpen-Einheit, dem Partikelsensor sowie einer Beruhigungsstrecke als Teil der Mess-Strecke angeordnet ist, wobei in Fluidströmungsrichtung gesehen die Motor-Pumpen-Einheit vor der Beruhigungsstrecke und die Beruhigungsstrecke vor dem Partikelsensor angeordnet sind, wobei, um eine Vorspannung des Fluids vor dem Partikelsensor und im Bereich desselben zu erzeugen, die Motor-Pumpen-Einheit das Fluid der Beruhigungsstrecke zuführt und ein einstellbares Druckbegrenzungsventil oder ein federbelastetes Rückschlagventil stromabwärts des Partikelsensors angeordnet ist, wobei die Probenaufbereitungs-Strecke vor und hinter den zwei Schaltventilen der Anschlusseinrichtung an die Mess-Strecke angeschlossen ist, so dass die Probenaufbereitungs-Strecke in einer Nebenleitung zu der Mess-Strecke angeordnet ist, so dass das Fluid bevorzugt durch die Mess-Strecke und die Probenaufbereitungs-Strecke einmalig oder mehrmalig im Kreis umlaufen und die Zuschaltung der Probenaufbereitungs-Strecke nach Bedarf erfolgen kann.

Weitere Partikelsensoren gehen aus der JP S62-255849, der DE 39 31 497 A1, der WO 2008/047559 A1 und der US 5 968 371 hervor.

Ausgehend von diesem Stand der Technik liegt der Erfindung die Aufgabe zugrunde, eine Vorrichtung zum Feststellen oder zum Messen von Partikelverschmutzungen in Fluiden derart weiter zu verbessern, dass eine zuverlässigere Feststellung bzw. Messung der Partikelverschmutzung ermöglicht ist.

Eine dahingehende Aufgabe löst eine Vorrichtung mit den Merkmalen des Patentanspruches 1 in seiner Gesamtheit.

Gemäß dem Kennzeichen von Anspruch 1 ist vorgesehen, dass die Einrichtung aus einem Probenbehälter besteht, der unter Beruhigung des aufgenommenen Fluids aus der Mess-Strecke dem Ausscheiden von gasförmigem Medium dient.

Dadurch, dass erfindungsgemäß eine Probenaufbereitungs-Strecke mittels einer Anschlusseinrichtung fluidführend an die Messstrecke anschließbar ist, und dass die Probenaufbereitungs-Strecke zumindest eine zusätzliche Einrichtung zum Feststellen der jeweils weiteren fluidfremden Verunreinigung und/oder zu deren zumindest teilweisem Ausscheiden aus dem Fluid aufweist, ist es möglich, den jeweils weiteren Fremdstoff aus dem Druckfluid des fluidischen Kreises der gesamthydraulischen Anlage einer getrennten Betrachtung zu unterziehen oder gar aus dem Druckfluid abzuscheiden, wohingegen vorzugsweise zeitgleich der eigentliche Partikelsensor weiter die Partikelverschmutzung im Druckfluid feststellt oder detektiert, ohne von den weiteren Fremdstoffen in der Feststell- oder Messqualität beeinträchtigt zu sein. Die sonst von dem Partikelsensor detektierten Fremdstoffe, wie beispielsweise Ölalterungsprodukte, Wasser oder Luftblasen, werden dann abgeschieden oder in Lösung gebracht und können insoweit auch nicht das Messergebnis betreffend den ungewollten Partikeleintrag nachteilig beeinflussen, weder was die Partikelanzahl anbelangt, noch deren etwaige Größenfeststellung. Da die genannten Fremdstoffe mithin nicht die Messung des Partikeleintrages nachteilig beeinflussen können, kann der Partikelsensor tatsächlich die jeweils vorherrschende Partikelverschmutzung feststellen, um dergestalt dann eine Aussage treffen zu können, ob ein Ersatz des Druckfluides gegen ein Neumedium notwendig ist und/oder ob zumindest an Teilen der gesamthydraulischen Anlage dergestalt Verbesserungen vorzunehmen sind, dass eben ein erhöhter Partikelverschmutzungseintrag, insbesondere bedingt durch abrasive Verschleißpartikel, erst gar nicht auftritt. Mit der erfindungsgemäßen Vorrichtung können auch feinste Verschmutzungen des Fluids detektiert werden, bei denen die Teilchen einen mittleren Durchmesser von größer als 14 *µ*m, bevorzugt größer als 6 *µ*m, weiter bevorzugt größer als 4 *µ*m, haben, vgl. hierzu auch ISO 4406. Ein weiterer Vorteil der Erfindung ist, dass auch dann Partikel erkannt werden, wenn sie die gleiche Dichte wie das Fluid haben.

Da die weiteren Fremdstoffe gleichfalls die Qualität des Druckfluids beeinträchtigen, kann bei einer bevorzugten Ausführungsform der erfindungsgemäßen Vorrichtung vorgesehen sein, dass mittels geeigneter Einrichtungen, die mit der Probenaufbereitungs-Strecke zusammenwirken, die Fremdstoffe zumindest teilweise auch aus dem Fluidkreis, sprich aus der Mess-Strecke, bleibend entfernt werden können.

Besonders bevorzugt besteht die jeweilige Einrichtung weiter aus:
- einer Filtereinrichtung, die den Fremdstoffeintrag im Fluid zumindest teilweise reduziert und filtergängige Medien zum Detektieren oder Ausscheiden derselben zumindest teilweise durchlässt, und/oder
- einer Temperiereinrichtung, d.h. einer Heizung und/oder einem Kühler, wobei die Temperiereinrichtung zum Auflösen oder Abscheiden von temperaturabhängigen Medien, wie Ölalterungsprodukten, mit temperaturabhängiger Löslichkeit dient.

Durch die jeweiligen Einrichtungen können die Eigenschaften des Fluids gezielt verändert werden, um möglichen Verfälschungen des Messergebnisses des Partikelsensors zu begegnen.

Der Probenbehälter kann dabei als Luftabscheider ausgeführt sein. Dabei können im Fluid vorhandene Luftbläschen im Luftabscheider langsam aufsteigen und über einen Luftablass entweichen, bis die im Fluid frei vorliegende Luftmenge ausreichend klein ist und die Messung nicht mehr verfälschen kann. Insbesondere zeigt der Partikelsensor bei dahingehender Luftabscheidung zunächst abfallende Werte an, die sich einem unteren Grenzwert annähern, der dann der tatsächlich im Druckfluid vorhandenen Partikelverschmutzung als dem einen Fremdstoff im Fluid entspricht.

Mit Hilfe einer zuschaltbaren Filtereinrichtung können in verschiedenen Anwendungsfällen die Messergebnisse gleichfalls verifiziert werden. Treten verschiedentlich, insbesondere bei Windkraftgetrieben, partikuläre Ölalterungsprodukte als Verschmutzung auf, würden diese bei der Partikelzählung mittels des Partikelsensors gleichfalls berücksichtigt werden und zu einer Verfälschung des Messergebnisses führen. Eine der Aufgaben der Vorrichtung sind die die Partikelzählung verfälschenden Einflüsse vorzugsweise zu eliminieren, zumindest aber zu reduzieren oder als Minimalforderung zumindest das Vorliegen einer Verfälschung als solche zu erkennen. Partikuläre Ölalterungsprodukte sind wie der Name sagt "Partikel" und gehören daher nicht zu einer Verfälschung; sehr wohl aber flüssige, insbesondere filtergängige Ölalterungsprodukte, die mithin eine Verfälschung der Partikelzählung bewirken. Eine solche Verfälschung kann dem Grunde nach nicht eliminiert werden; sie wird aber erkannt, wenn sich trotz einer zugeschalteten Filtration die Ölreinheit nicht oder nur minimal verbessert. Wenn sich die Ölreinheit bei zugeschalteter Filtration verbessert, aber auf einem mittleren Niveau "hängen" bleibt, erkennt man, dass eine Verfälschung gegeben ist, die aber regelmäßig noch vernachlässigt werden kann.

Eine andere Möglichkeit besteht auch darin, im Rahmen der Filtereinrichtung einen sogenannten Coalescer vorzusehen, der kleintropfige Wasseranteile zu großtropfigen zusammenführt, die dann aus dem Druckfluid in üblicher Weise durch eine Koalisiereinrichtung abgeführt werden können. Auch können über sogenannte Zyklonabscheider im Rahmen von konventionellen Filtersystemen Gasanteile aus dem Druckfluid abgetrennt werden, um dergestalt eine reine Detektierung der Feststoffverschmutzung mittels eines Partikelsensors vornehmen zu können.

Die Zuschaltung der Probenaufbereitungs-Strecke erfolgt nach Bedarf. Mithin ist es möglich, eine Fluidprobe aus einem System zu entnehmen und diese Probe über den Partikelsensor und die Einrichtung im Kreis zu fördern. Durch die permanente Umwälzung des Fluids wird gleichzeitig eine Sedimentation der Feststoffverschmutzung, insbesondere im Probenbehälter, verhindert. Es ist vorgesehen, nach Abschluss der Messungen den Probenbehälter zu leeren und erst anschließend einen neuen Messzyklus zu starten.

Weiterhin kann die Beruhigungsstrecke aus mindestens einem verlängerten Leitungsabschnitt besteht. Durch diese Beruhigungsstrecke können in begrenztem Umfang weitere Luftbläschen, die im Fluid bestehen, in Lösung gebracht werden, sodass diese die Partikelmessung nicht mehr verfälschen können.

In einer besonders vorteilhaften Ausführungsform können mehrere Einrichtungen in Reihe hintereinander geschaltet in der Probenaufbereitungs-Strecke angeordnet sein. Hierbei ist vorzugsweise in Fluidströmungsrichtung gesehen die Filtereinrichtung dem Probenbehälter vorgeschaltet. Dies ermöglicht es, das zu untersuchende Fluid in einem Durchlauf mehrfach zu modifizieren, insbesondere zu filtern und von Gasanteilen zu befreien. Auf diese Weise kann der angestrebte Grenzwert durch Abbau von das Messergebnis verfälschenden Bestandteilen des Fluids schneller erreicht und somit die Partikelmessung insgesamt beschleunigt und präzise durchgeführt werden.

Zur Gesamtüberwachung der Mess-Strecke sowie der Probenaufbereitungs-Strecke können Drucksensoren sowie zumindest ein Füllstandssensor dienen. Zum Vorspannen des Fluids sowie zur Abfuhr von fluidfremden Verunreinigungen können federbelastete Rückschlagventile vorgesehen sein. Die federbelasteten Rückschlagventile können dabei einstellbar ausgebildet sein. Die Drucksensoren ermöglichen es, einen schädigenden Überdruck in den Leitungen, insbesondere vor dem Partikelsensor, und im Luftabscheider zu verhindern. Durch den Füllstandssensor kann zudem die Schaltung der Anschlusseinrichtung bewirkt werden, sodass die Schaltventile der Anschlusseinrichtung abhängig vom Füllstand eine Überleitung von Fluid aus der Probenaufbereitungs-Strecke in die Mess-Strecke ermöglichen bzw. bei Entleerung einen Nachfluss von Fluid in die Probenaufbereitungs-Strecke verhindern. Durch ein stromabwärts des Partikelsensors angeordnetes Rückschlagventil kann eine Vorspannung des Fluids vor dem Partikelsensor und im Bereich desselben erreicht werden. Weiterhin kann der Luftabscheider mit Rückschlagventilen ausgestattet sein, um ein Nachsaugen von Luft bzw. ein Entweichen derselben im laufenden Betrieb zu ermöglichen. Stromauf des so gebildeten Nachsaugventils kann ein Belüftungsfilter angeordnet sein.

Besonders bevorzugt kann mittels einer zentralen Steuereinrichtung die Anschlusseinrichtung zum Zu- oder Wegschalten der Probenaufbereitungs-Strecke ansteuerbar sein. Insbesondere geben von der jeweiligen Einrichtung detektierte und/oder ausgeschiedene Medien die Zu- und Wegschaltzeit der Probenaufbereitungs-Strecke vor. Beispielsweise werden die Schaltventile der Ansteuereinrichtung aufgrund des im Probenbehälter gemessenen Füllstands oder aufgrund der mit den Drucksensoren erfassten Drücke in sinnfälliger Weise geschaltet.

Die Erfindung ist nachfolgend anhand von in den Figuren dargestellten Ausführungsbeispielen näher erläutert. Es zeigen:
- Fig. 1: eine erfindungsgemäße Vorrichtung zum Messen von Partikelverschmutzungen in Fluiden gemäß einer ersten Ausführungsform; und
- Fig. 2 bis 6: ausschnittsweise Darstellungen weiterer Ausführungsformen der Vorrichtung zum Messen von Partikeln in verschiedenen Betriebszuständen.

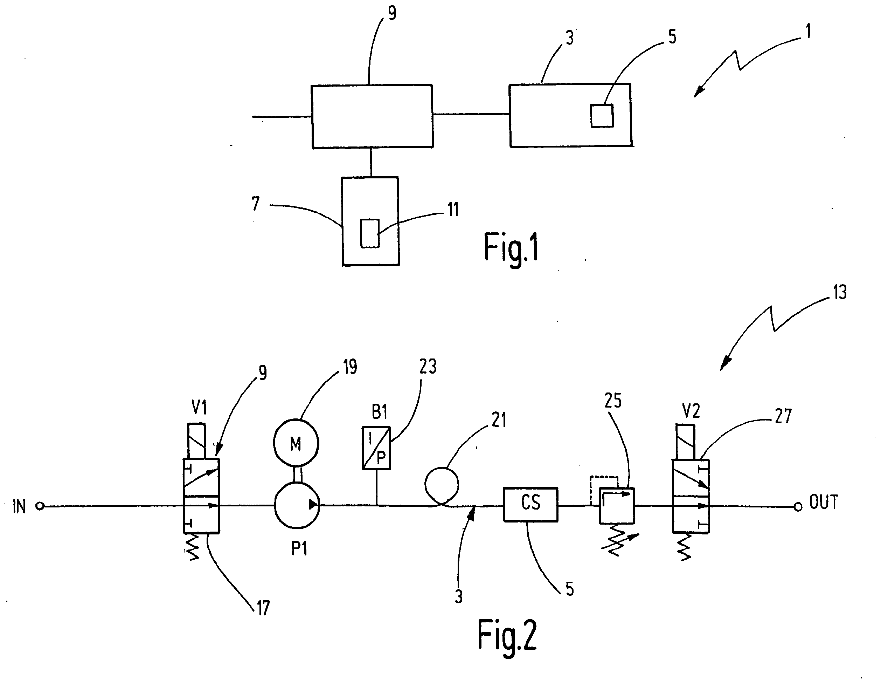

In der Fig. 1 ist eine erste Vorrichtung 1 zum Messen von Partikelverschmutzungen in Fluiden gezeigt. Die Fluide können neben einem Partikeleintrag weitere fluidfremde Verunreinigungen, wie flüssige, feste, gasförmige und/oder filtergängige und/oder temperaturabhängige Medien aufweisen, die mit der Vorrichtung zuverlässig erfasst werden. Die Vorrichtung 1 weist eine Mess-Strecke 3 mit einem Partikelsensor 5 zur Messung des Partikeleintrages in dem Fluid auf. Ferner ist eine Probenaufbereitungs-Strecke 7 vorgesehen, die mittels einer Anschlusseinrichtung 9 fluidführend an die Mess-Strecke 3 anschließbar ist. Die Probenaufbereitungs-Strecke weist zumindest eine zusätzliche Einrichtung 11 zum Detektieren der weiteren fluidfremden Verunreinigungen und/oder zu deren zumindest teilweisem Ausscheiden aus dem Fluid auf.

Die Einrichtung 11 kann aus einem Probenbehälter bestehen, der unter Beruhigung des aufgenommenen Fluids aus der Mess-Strecke 3 dem Ausscheiden von gasförmigen Medien dient. Alternativ oder zusätzlich kann eine (weitere) Einrichtung 11 aus einer Filtereinrichtung bestehen, die den Partikeleintrag im Fluid zumindest teilweise reduziert und filtergängige Medien zum Detektieren derselben zumindest teilweise durchlässt. Weiterhin kann eine Einrichtung 11 aus einer Temperiereinrichtung, das heißt einer Heizung oder einem Kühler, bestehen, wobei die Temperiereinrichtung zum Detektieren von temperaturabhängigen Medien, wie Ölalterungsprodukten, mit temperaturabhängiger Löslichkeit dient.

In Fig. 2 ist ein erster Ausschnitt aus einer Vorrichtung 13 gemäß einer zweiten Ausführungsform gezeigt, die eine Mess-Strecke 3 in Verbindung mit einer Anschlusseinrichtung 9 aufweist. Dieser Ausschnitt zeigt den Fluidweg im Normalbetrieb, das heißt bei vernachlässigbaren Verfälschungen durch flüssige Verunreinigungen etc. und einem begrenzten Anteil an Luftbläschen im Fluid. Die Mess-Strecke 3 weist einen Partikelsensor 5 (Contamination Sensor, CS) auf. Am Eingangs-Anschluss "IN" wird eine Probe eines Fluides, das analysiert werden soll, der Mess-Strecke 3 zugeführt. Über ein entsprechend geschaltetes erstes 3/2-Wege-Ventil 17 der Anschlusseinrichtung 9 wird das Fluid der Mess-Strecke 3 zugeführt. Um eine Vorspannung des Fluids im Bereich des Partikelsensors 5 zu erzeugen, ist eine Motor-Pumpen-Einheit 19 vorgesehen, die das Fluid einer Beruhigungsstrecke 21 zuführt. Die Beruhigungsstrecke 21 besteht aus einem verlängerten Leitungsabschnitt, dessen Länge in einer bevorzugten Ausführungsform sogar einstellbar sein kann. Ein in der Zeichnung gezeigter Drucksensor 23 ist dazu vorgesehen zu erkennen, ob Fluid oder Luft (Behälter leer) gefördert wird; so bricht der Druck bei Vorhandensein von Luft ein. Stromabwärts des Partikelsensors 5 ist ein einstellbares Druckbegrenzungsventil oder Rückschlagventil 25 vorgesehen. Weiter stromabwärts ist ein zweites 3/2-Wege-Ventil 27 der Anschlusseinrichtung 9 angeordnet, das ebenfalls entsprechend geschaltet ist, sodass das Fluid zum Fluid-Ausgangsanschluss "OUT" gelangen kann.

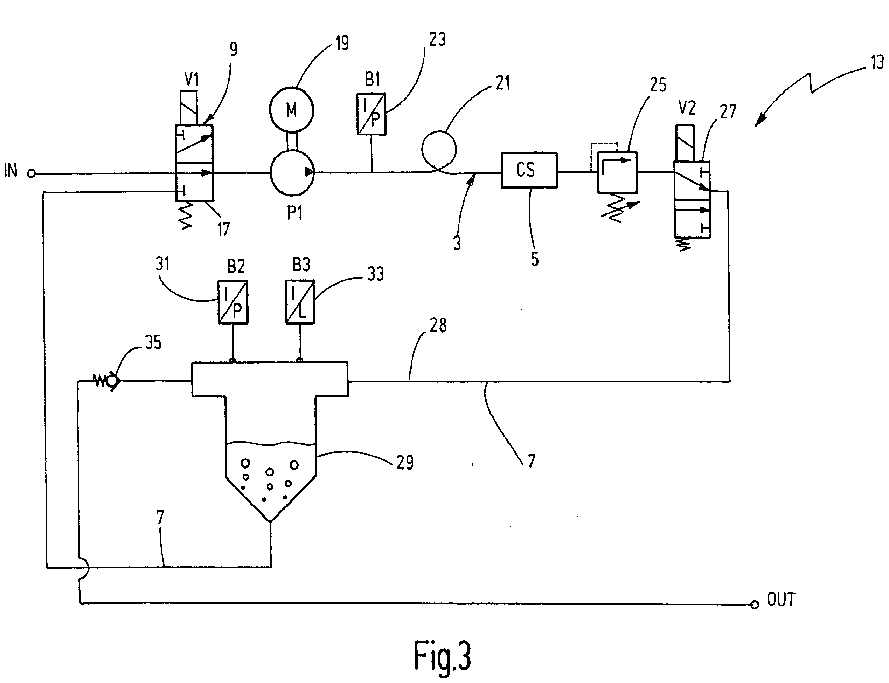

Die Fig. 3 zeigt eine erweiterte Darstellung der zweiten Ausführungsform. Das Fluid, das einmal die Mess-Strecke 3 durchlaufen hat, wird in einem vom Normalbetrieb abweichenden Sonderbetrieb statt zum Fluidausgang "OUT" durch das zweite 3/2-Wege-Ventil 27 der Anschlusseinrichtung 9 in eine Probenaufbereitungs-Strecke 7 übergeleitet. In der Probenaufbereitungs-Strecke 7, einer Nebenleitung 28 zur Mess-Strecke 3, wird das Fluid einem Probenbehälter 29 in Form eines Luftabscheiders zugeführt. Da das erste 3/2-Wege-Ventil 17 der Anschlusseinrichtung 9 in diesem Betriebszustand den Fluidanschluss "IN" mit der Mess-Strecke 3 verbindet, kann noch kein Fluid aus dem Probenbehälter 29 weiter fließen. Mithin sammelt sich das Fluid im Probenbehälter 29 an und beruhigt sich, so dass etwaig vorhandene Luftbläschen zur Oberfläche aufsteigen können. Ein Drucksensor 31 und ein Füllstandssensor 33 (auch als Niveau- oder Levelsensor bezeichnet) am Probenbehälter 29 sind zur Überwachung des Probenbehälters 29 vorgesehen. Der genannte Füllstandssensor ist nicht unbedingt erforderlich, trägt aber zur Betriebssicherheit wesentlich mit bei. Eine Füllstandsüberwachung könnte dem Grunde nach auch über den Druck im Behälter 31 und den Druck nach der Pumpe 19 erfolgen. Sollte der Luftdruck oder der Fluiddruck im Probenbehälter 29 zu groß werden, kann die Luft oder das Fluid über ein federbeaufschlagtes Rückschlagventil 35 zum Fluidausgang "OUT" entweichen.

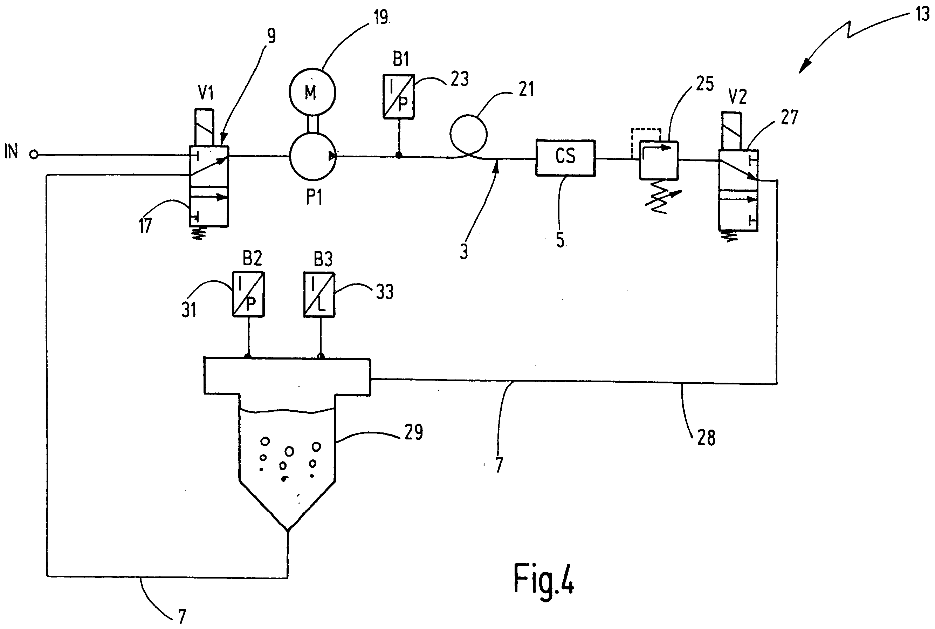

Wenn der Füllstand im Probenbehälter 29 ein für die Durchführung der Messung hinreichendes Maß erreicht hat, schaltet das erste 3/2-Wege-Ventil 17 um, wie es in Fig. 4 gezeigt ist, und ermöglicht es, das Fluid mit Hilfe der Motor-Pumpen-Einheit 19 durch die Mess-Strecke 3 und die Probenaufbereitungs-Strecke 7 im Kreis zu fördern. Die Anzahl der Partikel, welche ausschlaggebend für die Verschmutzung ist, wird durch den Partikelsensor 5 ermittelt. Etwaige Luftbläschen, die nicht schon in der Beruhigungsstrecke 21 in Lösung gegangen sind, können bei jedem Durchfluss des Fluids durch den Probenbehälter 29 aus dem Fluid entweichen und werden somit abgeschieden. Dementsprechend vermindert sich die Anzahl der Luftbläschen im Fluid und der Partikelsensor 5 stellt über die Zeit eine abnehmende Partikelanzahl fest, da immer weniger Luftbläschen detektiert werden. Nach einem oder mehreren Umläufen des Fluids durch die Mess-Strecke 3 und die Probenaufbereitungs-Strecke 7 nähert sich die Anzahl der gemessenen Partikel einem unteren Grenzwert an, der die wahre Partikeleintragrate wiedergibt.

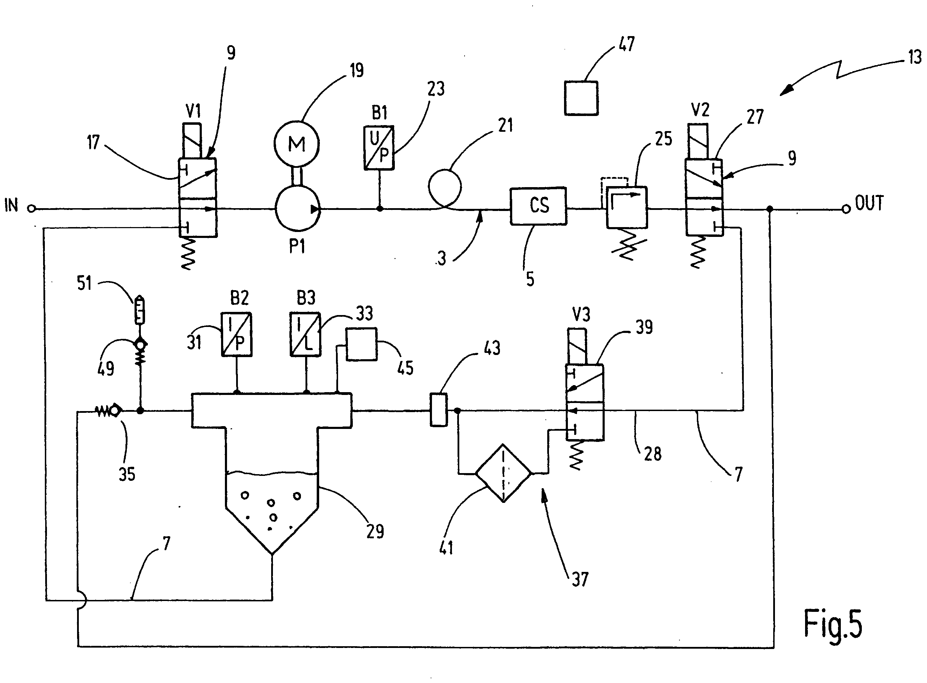

In der Fig. 5 ist ein vervollständigter Hydraulikplan der Vorrichtung 13 gemäß der zweiten Ausführungsform gezeigt. Diese weist in der Probenaufbereitungs-Strecke 7 stromaufwärts des Probenbehälters 29 eine Filtereinrichtung 37 mit einem durch ein 3/2-Wege-Ventil 39 zuschaltbaren Filter 41 auf. Durch die zuschaltbare Filtereinrichtung 37 kann ermittelt werden, ob in einem Fluid herausfilterbare oder filtergängige, insbesondere flüssige, Verunreinigungen enthalten sind. Wenn die Partikelanzahl im Fluid nach dem Durchfluss durch den Filter 41 absinkt, handelt es sich um herausfilterbare Partikel. Andernfalls ist von filtergängigen Verunreinigungen auszugehen. Darüber hinaus ist die Filtereinrichtung 37 zur Reinigung der Vorrichtung 13 vorgesehen.

Zwischen der zuschaltbaren Filtereinrichtung 37 und dem Probenbehälter 29 ist zudem eine Temperiereinrichtung 43 zur Detektion von Ölalterungsprodukten mit temperaturabhängiger Löslichkeit, wie z.B. Varnish, vorgesehen. Durch das Aufheizen des Fluids kann eine Lösung der flüssigen Verunreinigungen im Fluid bewirkt werden. Die Temperiereinrichtung 43 kann aber auch als Kühler betrieben werden, um flüssige Verunreinigungen aus dem Fluid abzuscheiden und diese somit mit dem Partikelsensor 5 detektierbar zu machen. Mithin werden bei abnehmender Öltemperatur steigende Zählraten am Partikelsensor 5 festgestellt und einer entsprechenden Auswertung durch die Auswertelektronik unterzogen.

Weiterhin kann in der Probenaufbereitungs-Strecke 7 ein Vakuumgenerator 45 vorgesehen sein. Der Vakuumgenerator 45 dient der Extraktion von gasförmigen Bestandteilen im Fluid. Diese können dann im stromabwärts gelegenen Probenbehälter 29 abgeschieden werden (= Mess- und Auswertelektronik).

Eine prinzipiell in Fig. 5 dargestellte Steuereinrichtung 47 ist zur Steuerung des Gesamtablaufs vorgesehen. Diese ist mit den Drucksensoren 23, 31 und dem Füllstandssensor 33 sowie der Anschlusseinrichtung 9 gekoppelt. Ferner kann der Partikelsensor 5 an die Steuereinrichtung 47 angeschlossen sein.

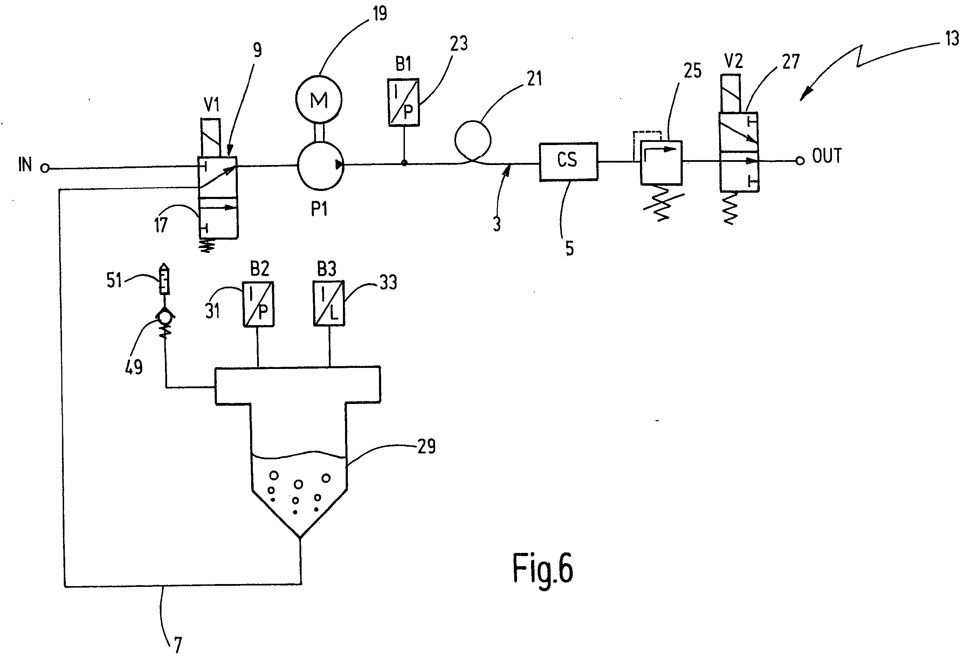

Nach Abschluss der Messung wird die Anschlusseinrichtung 9 so geschaltet (vgl. Fig. 6), dass das zweite 3/2-Wege-Ventil 27 das Fluid aus der MessStrecke 3 dem Fluidausgang "OUT" zuleitet. Auf diese Weise wird der Probenbehälter 29 entleert und für die Aufnahme einer neuen Fluidmenge vorbereitet. Hierbei kann Luft durch ein Rückschlagventil 49 und einen Belüftungsfilter 51 nachströmen.

Durch die erfindungsgemäßen Ausführungsformen werden Vorrichtungen 1, 13 zum Messen von Partikelverschmutzungen in Fluiden aufgezeigt, die es ermöglichen, feste, flüssige, gasförmige-, filtergängige und/oder temperaturabhängige Verunreinigungen, die das Messergebnis verfälschen können, weitgehend zu eliminieren. Insbesondere kann auch bei einem hohen Luftgehalt im Fluid eine zuverlässige Messung der Partikel im Fluid durchgeführt werden.

Über den Fluideingang "IN" und den Fluidausgang "OUT" lässt sich die erfindungsgemäße Vorrichtung in hydraulische Gesamtsysteme, wie Windkraftanlagen, Pressen, Transferstraßen etc. einbinden, so dass ein Online-Messbetrieb mittels des Partikelsensors 5 möglich ist. Mit der erfindungsgemäßen Lösung ist es möglich, Messwert-Verfälschungen, auch wenn sie nicht immer eliminiert werden können, zumindest als solche messtechnisch zu erkennen und bei der Auswertung entsprechend zu berücksichtigen.

## Patentansprüche

1. Vorrichtung zum Feststellen von Fremdstoffen, wie Partikeln, in Fluiden, die neben diesem Partikeleintrag weitere fluidfremde Verunreinigungen, wie feste, flüssige und gasförmige Fremdstoffe, aufweisen können, mit einer Mess-Strecke (3), die einen Partikelsensor (5) zum Feststellen des Partikeleintrages aufweist, wobei eine Probenaufbereitungs-Strecke (7) mittels einer Anschlusseinrichtung (9) fluidführend an die Mess-Strecke (3) anschließbar ist, wobei die Probenaufbereitungs-Strecke (7) zumindest eine zusätzliche Einrichtung (11, 29, 37, 43, 45) zum Feststellen der jeweils weiteren fluidfremden Verunreinigung und/oder zu deren zumindest teilweisem Ausscheiden aus dem Fluid aufweist, wobei die Anschlusseinrichtung (9) aus elektromagnetisch ansteuerbaren Schaltventilen (17, 27), insbesondere in Form von 3/2-Wege-Ventilen, gebildet ist, und wobei zwischen zwei Schaltventilen (17, 27) der Anschlusseinrichtung (9) eine Baugruppe bestehend aus einer Motor-Pumpen-Einheit (19), dem Partikelsensor (5) sowie einer Beruhigungsstrecke (21) als Teil der Mess-Strecke (3) angeordnet ist, wobei in Fluidströmungsrichtung gesehen die Motor-Pumpen-Einheit (19) vor der Beruhigungsstrecke (21) und die Beruhigungsstrecke (21) vor dem Partikelsensor (5) angeordnet sind, wobei, um eine Vorspannung des Fluids vor dem Partikelsensor (5) und im Bereich desselben zu erzeugen, die Motor-Pumpen-Einheit (19) das Fluid der Beruhigungsstrecke (21) zuführt und ein einstellbares Druckbegrenzungsventil oder ein federbelastetes Rückschlagventil (25) stromabwärts des Partikelsensors (5) angeordnet ist, wobei die Probenaufbereitungs-Strecke (7) vor und hinter den zwei Schaltventilen (17, 27) der Anschlusseinrichtung (9) an die Mess-Strecke (3) angeschlossen ist, so dass die Probenaufbereitungs-Strecke (7) in einer Nebenleitung (28) zu der Mess-Strecke (3) angeordnet ist, so dass das Fluid bevorzugt durch die Mess-Strecke (3) und die Probenaufbereitungs-Strecke (7) einmalig oder mehrmalig im Kreis umlaufen und die Zuschaltung der Probenaufbereitungs-Strecke (7) nach Bedarf erfolgen kann, **dadurch gekennzeichnet, dass** die Einrichtung (11) aus einem Probenbehälter (29) besteht, der unter Beruhigung des aufgenommenen Fluids aus der Mess-Strecke (3) dem Ausscheiden von gasförmigem Medium dient.

2. Vorrichtung nach Anspruch 1, **dadurch gekennzeichnet, dass** die jeweilige Einrichtung (11) weiter
- aus einer Filtereinrichtung (37) besteht, die den Fremdstoffeintrag im Fluid zumindest teilweise reduziert und filtergängige Medien zum Detektieren oder Ausscheiden derselben aus dem Fluid zumindest teilweise durchlässt, und/oder
- aus einer Temperiereinrichtung (43) (Heizung und/oder Kühler) besteht, die zum Detektieren oder Auflösen oder Abscheiden von temperaturabhängigen Medien, wie Ölalterungsprodukten, mit temperaturabhängiger Löslichkeit dient.

3. Vorrichtung Anspruch 1 oder 2, **dadurch gekennzeichnet, dass** die Beruhigungsstrecke (21) aus mindestens einem verlängerten Leitungsabschnitt besteht.

4. Vorrichtung nach Anspruch 2 oder 3, **dadurch gekennzeichnet, dass** mehrere Einrichtungen (11, 29, 37, 43, 45), in Reihe hintereinandergeschaltet, Teil der Probenaufbereitungs-Strecke (7) sind und dass vorzugsweise in Fluidströmungsrichtung gesehen die Filtereinrichtung (37) dem Probenbehälter (29) vorgeschaltet ist.

5. Vorrichtung nach einem der vorhergehenden Ansprüche, **dadurch gekennzeichnet, dass** zur Gesamtüberwachung von Mess-Strecke sowie Probenaufbereitungs-Strecke (7) Drucksensoren (23, 31) sowie zumindest ein Füllstandssensor (33) dienen und dass zum Vorspannen des Fluidkreises (3, 7) oder zur Abfuhr von fluidfremden Verunreinigungen federbelastete Rückschlagventile (25, 35) dienen.

6. Vorrichtung nach einem der vorhergehenden Ansprüche, **dadurch gekennzeichnet, dass** mittels einer zentralen Steuereinrichtung (47) die Anschlusseinrichtung (9) zum Zu- und Wegschalten der Probenaufbereitungs-Strecke (7) ansteuerbar ist und dass insbesondere von der jeweiligen Einrichtung (11, 29) detektierte Medien die Zu- und Wegschaltzeit der Probenaufbereitungs-Strecke (7) vorgeben.

## Claims

1. A device for determining foreign substances, such as particles, in fluids, which may, in addition to this amount of particles, contain further impurities which are foreign to the fluid, such as solid, liquid and gaseous foreign substances, with a measurement section (3) which has a particle sensor (5) for determining the amount of particles, wherein a sample preparation section (7) can be connected fluidically to the measurement section (3) by means of a connecting device (9), wherein the sample preparation section (7) has at least one additional device (11, 29, 37, 43, 45) for determining the respective impurity which is foreign to the fluid, and/or for at least partial deposition thereof from the fluid, wherein the connecting device (9) is formed from electromagnetically actuatable shift valves (17, 27), in particular in the form of 3/2 way shift valves, and wherein, an assembly, consisting of a motor pump unit (19), the particle sensor (5) as well as a stilling section (21) is arranged between two shift valves (17, 27) of the connecting device (9) as part of the measurement section (3), wherein, seen in fluid flow direction, the motor pump unit (19) is arranged in front of the stilling section (21) and the stilling section (21) is arranged in front of the particle sensor (5), wherein, in order to produce a preloading of the fluid before the particle sensor (5) and in the region of same, the motor pump unit (19) supplies the fluid to the stilling section (21) and an adjustable pressure limiting valve or a spring-loaded non-return valve (25) is arranged upstream of the particle sensor (5), wherein the sample preparation section (7) is connected to the measurement section (3) in front of and behind the two shift valves (17, 27) of the connection device (9), with the result that the sample preparation section (7) is arranged in a secondary feed line (28) to the measurement section (3), with the result that the fluid can circulate once or several times in the circuit, preferably through the measurement section (3) and the sample preparation section (7), and the switch to the sample preparation section (7) can take place as required, **characterised in that** the device (11) consists of a sample container (29) which serves to precipitate gaseous medium by stilling the accommodated fluid from the measurement section (3).

2. The device according to claim 1, **characterised in that** the respective device (11) also
- consists of a filter device (37) which at least partly reduces the amount of foreign substances in the fluid and at least partly allows through filterable media for detecting or precipitating same out of the fluid, and/or
- consists of a temperature-controlling device (43) (heater and/or cooler) which serves to detect or dissolve or precipitate out temperature-dependent media, such as oil-ageing products, with temperature-dependent solubility.

3. The device according to claim 1 or 2, **characterised in that** the stilling section (21) consists of at least one elongated line section.

4. The device according to claim 2 or 3, **characterised in that** several devices (11, 29, 37, 43, 45), arranged in series one after the other, are part of the sample preparation section (7) and that, seen preferably in fluid flow direction, the filter device (37) is connected upstream of the sample container (29).

5. The device according to one of the preceding claims, **characterised in that** pressure sensors (23, 31) as well as at least one fill level sensor (33) serve for overall monitoring of measuring section as well as sample preparation section (7), and that spring-loaded non-return valves (25, 35) serve to preload the fluid circuit (3, 7) or to remove impurities which are foreign to the fluid.

6. The device according to one of the preceding claims, **characterised in that** the connecting device (9) for connecting and removing the sample preparation section (7) can be actuated by means of a central control device (47) and that media detected in particular by the respective device (11, 29) affect the connection and removal time of the sample preparation section (7).

## Revendications

1. Installation de constatation de substances étrangères comme des particules dans des fluides qui, outre ces particules entraînées, peuvent comporter d'autres impuretés étrangères aux fluides, comme des substances solides, liquides ou gazeuses, comprenant une section (3) de mesure qui a un capteur (5) de particule pour la constatation des particules entraînées, dans laquelle une section (7) de préparation d'échantillon peut être raccordée fluidiquement à la section (3) de mesure au moyen d'un dispositif d'un dispositif (9) de raccordement, la section (7) de préparation d'échantillon ayant au moins un dispositif (11, 29, 37, 43, 45) supplémentaire pour la constatation de l'autre impureté étrangère au fluide et/ou pour sa séparation au moins partielle du fluide, le dispositif (9) de raccordement étant formé de vannes (17, 27) de commande pouvant être commandées électromagnétiquement, notamment sous la forme de vannes à 3/2 voies et, entre deux vannes (17, 27) de commande du dispositif (9) de raccordement, est monté un module constitué d'un groupe (19) motopompe du capteur (5) de particule ainsi que d'une section (21) de tranquillisation comme partie de la section (3) de mesure, dans lequel, considéré dans le sens de passage du fluide, le groupe (9) motopompe est disposé avant la section (21) de tranquillisation et la section (21) de tranquillisation est disposée avant le capteur (5) de particule, dans lequel, pour produire une pré-compression du fluide avant le capteur (5) de particule et dans la région de celui-ci, le groupe (19) motopompe envoie le fluide à la section (21) de tranquillisation et une soupape de limitation de la pression réglable ou un clapet antiretour (25) soumis à l'action d'un ressort est monté en aval du capteur (5) de particule, la section (7) de préparation d'échantillon étant raccordée à la section (3) de mesure avant et derrière les deux vannes (17, 27) de commande du dispositif (9) de raccordement de manière à ce que la section (7) de préparation d'échantillon soit disposée dans un conduit (28) auxiliaire par rapport à la section (3) de mesure, de sorte que le fluide circule en circuit une fois ou plusieurs fois, de préférence dans la section (3) de mesure et la section (7) de préparation d'échantillon et de manière à ce que le branchement de la section (7) de préparation d'échantillon puisse s'effectuer suivant les besoins, **caractérisée en ce que** le dispositif (11) est constitué d'un récipient (29) à échantillon qui, en tranquillisant le fluide reçu de la section (3) de mesure, sert à séparer du fluide gazeux.

2. Installation suivant la revendication 1, **caractérisée en ce que** le dispositif (11) respectif, en outre
- est constitué d'un dispositif (37) de filtration qui réduit, au moins en partie, l'entraînement de substances étrangères dans le fluide et laisse passer, au moins en partie, des fluides passant dans le filtre pour leur détection ou leur séparation du fluide, et/ou
- est constitué d'un dispositif (43) de mise en température (chauffage et/ou refroidissement) qui sert, par une solubilité qui dépend de la température, à détecter ou à décomposer ou à séparer des fluides en fonction de la température, comme des produits de vieillissement d'huile.

3. Installation suivant la revendication 1 ou 2, **caractérisée en ce que** la section (21) de tranquillisation est constituée d'au moins un tronçon de conduit rallongé.

4. Installation suivant la revendication 2 ou 3, **caractérisée en ce que** plusieurs dispositifs (11, 29, 37, 43, 45) montés en série les uns derrière les autres font partie de la section (7) de préparation d'échantillon et **en ce que**, de préférence, considéré dans le sens de passage du fluide, le dispositif (37) de filtration est en amont du réservoir (29) d'échantillon.

5. Installation suivant l'une des revendications précédentes, **caractérisée en ce que** des capteurs (23, 31) de pression ainsi qu'au moins un capteur (33) de niveau servent au contrôle d'ensemble de la section de mesure ainsi que de la section (7) de préparation d'échantillon et **en ce que** des clapets antiretour (25, 35) soumis à l'action d'un ressort servent à la pré-compression du circuit (3, 7) de fluide ou à l'évacuation d'impuretés étrangères au fluide.

6. Installation suivant l'une des revendications précédentes, **caractérisée en ce que** le dispositif (9) de raccordement peut, au moyen d'un dispositif (47) central de commande, être commandé pour le branchement et le débranchement de la section (7) de préparation d'échantillon et **en ce que** notamment des fluides détectés par le dispositif (11, 29) respectif prescrivent le temps de branchement et de débranchement de la section (7) de préparation d'échantillon.
